Europäisches Patentamt

European Patent Office

Office européen des brevets

(19)

(11) Numéro de publication: **0 247 991**

**A1**

(12) # DEMANDE DE BREVET EUROPEEN

(21) Numéro de dépôt: **87870074.9**

(22) Date de dépôt: **22.05.87**

(51) Int. Cl.⁴: **A 61 B 5/00**
**A 61 B 5/04, H 03 F 3/45**

(30) Priorité: **27.05.86 BE 216706**

(43) Date de publication de la demande:
**02.12.87 Bulletin 87/49**

(84) Etats contractants désignés:
**AT BE CH DE ES FR GB IT LI NL SE**

(71) Demandeur: **DELIMER S.A.**
**Rue de la Station 134**
**B-7700 Mouscron (BE)**

(72) Inventeur: **Delbeke, Jean, Dr.**
**Rue des Brasseurs 21**
**B-7700 Mouscron (BE)**

(74) Mandataire: **Dopchie, Jean-Marc**
**Kortrijks Octrooi- en Merkenbureau - K.O.B.**
**Conservatoriumplein 8**
**B-8500 Kortrijk (BE)**

(54) **Méthode et appareillage de captation multicanal pour l'enregistrement digital de signaux électrophysiologiques lents et de faible amplitude.**

(57) Méthode de captation multicanale pour l'enregistrement digital de signaux lents et de faible amplitude, captés au moyen de parties d'électrodes (500 et 600) formant les entrées de chaque canal d'une unité périphérique (100) et transmis par une ligne de liaison vers une unité centrale (200); caractérisée par une interrogation séquentielle des canaux qui fournissant un échantillon différentiel; par la transmission d'un marqueur d'événement; par une préamplification différentielle symétrique avec contrôle de l'état des électrodes; par l'avancement des échantillons dirigé par des trains d'impulsions venant de l'unité centrale et par une correction continue des échantillons pendant les temps d'attente.

FIG1

EP 0 247 991 A1

## Description

"Méthode et appareillage de captation multicanal pour l'enregistrement digital de signaux électrophysiologiques lents et de faible amplitude".

L'invention concerne une méthode et un appareillage de captation multicanal pour l'enregistrement digital de signaux électrophysiologiques lents et de faible amplitude.

Les signaux électrophysiologiques sont le plus souvent obtenus par les électrodes placées au contact de l'organisme à examiner.

Ces signaux consistent en des variations de potentiel électrique de très faible amplitude, jusqu'à moins de 10 microvolts. Ils sont superposés à des potentiels continus d'origines diverses et d'amplitude beaucoup plus élevée jusqu'à 50 millivolts.

Dans le but de le rendre moins sensible aux interférences lors de la transmission vers une unité centrale, le signal est amplifié et parfois conditionné à proximité du lieu de sa captation par une unité périphérique.

Plusieurs canaux de mesure sont placés en parallèle. Pour des raisons de sécurité, une isolation galvanique est placée entre les électrodes de mesure et l'unité centrale de réception.

La très faible amplitude des signaux et les défauts de contact des électrodes de mesure imposent une impédance d'entrée et une réjection de mode commun élevées. Dans les appareillages généralement utilisés, on obtient cet effet au moyen de préamplificateurs d'entrée différentiels comportant pour chacune des deux électrodes du canal considéré un transistor à effet de champ. Pour l'un des deux transistors la source est reliée à une source de courant, controlée par un niveau de référence, pour l'autre la source est reliée à une source de courant contrôlée par la sortie d'un amplificateur dont les entrées sont connectées aux sorties des deux transistors à effet de champ, le gain étant réglé par une résistance en shunt entre les deux sources de courant. Comme le niveau de référence est constitué par la masse, ce type d'appareillage n'est pas parfaitement symétrique.

Pour éviter les nombreuses difficultés de la mesure, les appareillages actuellement utilisés comprennent un circuit permettant d'évaluer l'impédance de contact des électrodes, et comprennent généralement un sélecteur d'entrée permettant de relier les électrodes soit à un impédance-mètre, soit à un préamplificateur.

Dans une autre solution connue, l'impédance est surveillée de manière continue au moyen d'un courant injecté dans les électrodes à une fréquence nettement plus élevée que le signal à mesurer de façon à éviter les interférences avec ce signal.

Pour mesurer l'impédance en continu dans une bande de fréquence correspondante à celle du signal, on peut injecter un signal en mode commun, mais ceci à pour effet de réduire la réjection de mode commun.

Les divers appareillages ont le défaut d'être insensibles à un potentiel de polarisation des électrodes, qui peut perturber fortement les enregistrements des signaux lents, de fréquence inférieure au Hertz.

De même lorsqu'on place un condensateur de blocage à l'entrée des préamplifica teurs, les constantes de temps deviennent très élevées pour des signaux lents, et des phénomènes de saturation se produisent qui exigent des systèmes complexes de prévention.

Une alternative est de pourvoir chaque voie d'amplification continue d'une source de tension de compensation réglable à l'entrée, mais le réglage nécessaire rend le système peu pratique et complexe.

Dans la captation multicanale de signaux électrophysiologiques, la multiplicité des canaux impose un multiplexage d'échantillons successifs de chacun d'entre eux.

Ce multiplexage, qu'il soit fait au niveau du signal analogique ou au niveau d'un signal préalablement digitalisé est effectué par un circuit interrogeant successivement les différents canaux. La logique de contrôle de ce multiplexage doit être prévue pour un nombre déterminé de canaux et ce nombre ne peut être modifié.

Dans les appareils actuels, le signal analogique subit généralement une transformation digitale avant d'être traité par l'unité centrale. L'existence de circuits convertisseurs analogiques-digitaux miniaturisés et possédant une sortie série permet d'effectuer cette transformation au niveau de l'unité périphérique et simplifie l'isolation galvanique et la transmission des données, puisque les lignes sont moins nombreuses et que les données digitales sont moins sensibles aux sources d'imperfection et d'interférences.

Néanmoins les contrôles des filtres d'entrée, du multiplexeur, du circuit d'échantillonage, et du convertisseur analogique-digital rendent la transmission compliquée et alourdissent les circuits de l'unité périphérique. Ils augmentent sa consommation et diminuent la qualité de l'isolation.

Certains appareillages de télémétrie ont supprimé ces contrôles provenant de l'unité centrale, en transmet tant continuellement et de façon séquentielle le signal de chaque canal. Dans ce cas, l'unité périphérique est encore plus complexe puisqu'elle doit posséder une horloge précise. De plus, l'unité centrale perd tout contrôle des vitesses de transmission et d'échantillonage. Une modification de ces paramètres doit être effectuée au niveau de l'unité de transmission ce qui en limite les applications.

Il existe actuellement divers modes de transmission série controlée par une unité centrale. Ceux qui font appel à un nombre minimum de connections transportent les signaux de transmission et de réception soit sur deux lignes différentes soit en n'utilisant qu'une seule ligne en effectuant successivement la transmission dans un sens puis dans l'autre. Cette dernière solution est la plus parcimonieuse en lignes mais est plus complexe au niveau des circuits de contrôle.

Dans aucun de ces appareillages un événement inattendu dans les séquences des transmission ne peut être communiqué de l'unité périphérique vers l'unité centrale.

La méthode et l'appareillage de captation multicanal de signaux électrophysiologiques suivant l'invention a pour but de remédier aux divers inconvénients des appareillages actuels.

En particulier pour les signaux lents l'appareillage suivant l'invention possède une réjection de mode commun élevée, et permet l'évaluation continue de la qualité du contact des électrodes.

L'appareillage suivant l'invention peut être étendu à un nombre variable de canaux d'entrées et peut travailler en parallèle avec d'autres appareillages semblables.

L'appareillage suivant l'invention possède une communication simple entre l'unité périphérique de captation et l'unité centrale d'enregistrement de façon à permettre une isolation galvanique efficace et une grande mobilité de l'unité périphérique.

L'appareillage suivant l'invention permet un contrôle aisé au niveau de l'unité centrale des fréquences de transmission et des fréquences d'échantillonage tout en maintenant une seule ligne de transmission.

L'appareillage suivant l'invention transmet un signal marqueur d'événements de l'unité périphérique à l'unité centrale sans que l'unité périphérique ne soit interrogée au préalable par l'unité centrale.

La méthode de caption multicanal pour l'enregistrement digital de signaux électrophysiologiques lents et de faible amplitude, captés au moyen d'électrodes formant deux par deux les entrées de chaque canal d'une unité périphérique de captation et transmis vers une unité centrale d'enregistrement et de commande, suivant l'invention est caractérisée en ce que les canaux utilisés fournissent à tour de rôle un échantillon, représentant la différence d'amplitude du signal conditionné du canal en question tel qu'il se présente au moment où il est interrogé par rapport au moment de l'interrogation précédente, suivi d'une transmission sérielle des échantillons consécutifs.

La méthode de captation multicanal suivant l'invention, dans laquelle les deux entrées de chaque canal sont préamplifiées par un élément amplificateur propre, est en outre caractérisée en ce que chacun des dits éléments amplificateurs est contrôlé par une boucle de rétroaction différente pour chacun d'entre eux: la première boucle étant commandée par l'amplitude du signal différentiel des deux entrées, la deuxième boucle étant commandée par la tension de mode commun des deux entrées.

La méthode de captation multicanal suivant l'invention est en outre caractérisé en ce que la sélection des canaux utilisés, la transmission sérielle et la reconvertion en parallèle des échantillons sont contrôlées par des trains d'impulsions.

La méthode de captation multicanal suivant l'invention est en outre caractérisée en ce que le temps entre les trains d'impulsions est utilisé pour transmettre des signaux de marqueur d'événements.

D'autres caractéristiques et propriétés de la méthode et de l'appareillage de captation multicanal pour l'enregistrement digital de signaux électrophysiologiques lents et de faible amplitude, suivant l'invention, ressortiront de la description d'un tel appareillage illustrée au moyen des figures en annexe:

La figure 1 est un schéma synoptique de l'appareillage suivant l'invention.

La figure 2 est le schéma du circuit conditionnant l'entrée d'un canal de l'unité périphérique d'un tel appareillage de captation.

La figure 3 est le schéma du circuit d'échantillonage séquentiel et différentiel d'un canal de l'unité périphérique et du circuit d'initialisation d'un tel appareillage de captation.

La figure 4 est le schéma de l'unité centrale et de l'unité de transmission, dont une première partie est incluée dans l'unité périphérique, et la seconde forme l'entrée de l'unité centrale, d'un tel appareillage de captation.

La figure 5 est un diagramme montrant l'évolution au cours du temps de différents signaux suivant un mode particulier de captation de signaux électrophysiologiques suivant l'invention.

La figure 6 est le schéma de l'unité périphérique d'un appareillage de captation à deux canaux suivant l'invention.

La figure 7 est le schéma de l'unité centrale d'une appareillage de captation à deux canaux suivant l'invention.

La figure 8 est un diagramme logique expliquant la procédure avec laquelle le microprocesseur compense toutes les sources de dérive.

La figure 9 est le schéma du circuit d'échantillonnage séquentiel et différentiel d'un canal de l'unité périphérique et du circuit d'initialisation d'un autre exemple de réalisation d'un tel appareillage de captation.

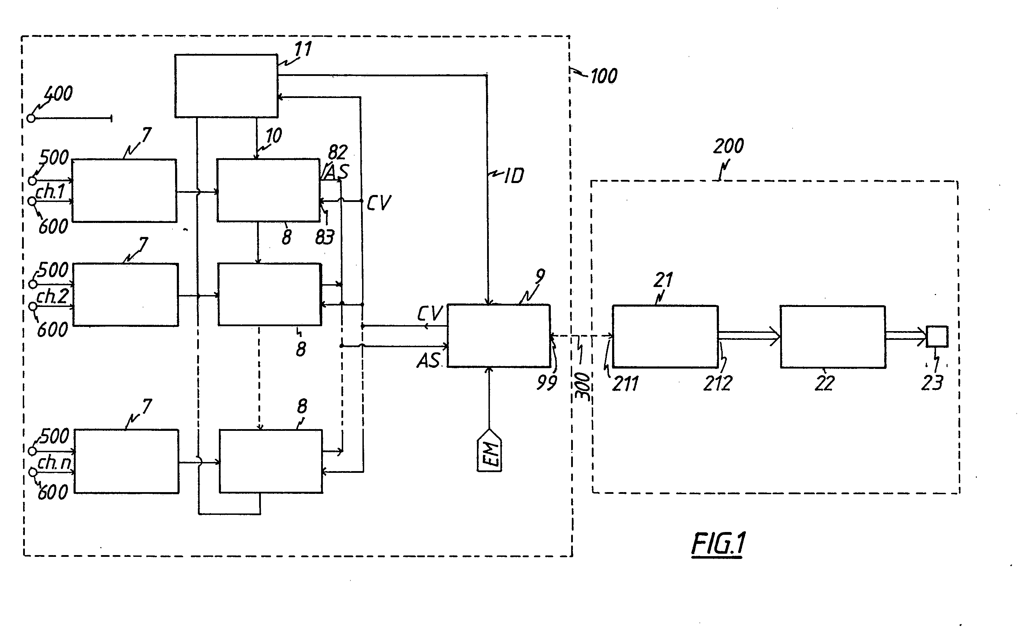

Dans un appareillage de captation multicanal suivant l'invention, fig. 1, on distingue une unité périphérique (100) de captation proprement dite et une unité centrale (200) d'enregistrement et de commande, ces deux unités étant reliées entre elles par une ligne (300) de liaison.

L'unité périphérique (100) est reliée à l'organisme examiné par une connection de masse (400) et plusieurs paires d'électrodes (500 et 600) de mesure formant les différents canaux de mesure. Le signal de chaque canal, capté par une paire d'électrodes (500 et 600), passe par un circuit de conditionnement (7) avant d'atteindre le circuit d'échantillonnage (8) différentiel et séquentiel.

A leur sortie (82), les circuits d'échantillonnage (8) sont reliés en étoile au circuit de transmission (9) de l'unité périphérique (100) pour les signaux de mesure (AS) et sont reliés mutuellement par une connection circulaire (10) interrompue par un circuit d'initialisation (11). Le circuit de transmission (9) périphérique est relié en étoile à l'entrée (83) des circuits d'échantillonnage (8) et au circuit d'initialisation (11) pour le signal de contrôle (CV). Le circuit de transmission (9) périphérique reçoit un signal de marquage d'événement (EM) en plus d'un signal d'identification (ID) venant du circuit d'initialisation (11).

La sortie (99) du circuit de transmission (9) périphérique, qui constitue la sortie de l'unité périphérique (100), est liée par la ligne de liaison (300) à l'entrée (211) de l'unité centrale (200) d'enregistrement qui coïncide avec l'entrée du circuit de transmission (21) central dont la sortie (212) est reliée à un interface (22) composé de décodeurs d'adresse et de circuits "latch" de façon à rendre les données directement accessibles à un microprocesseur au niveau de son "bus" (23).

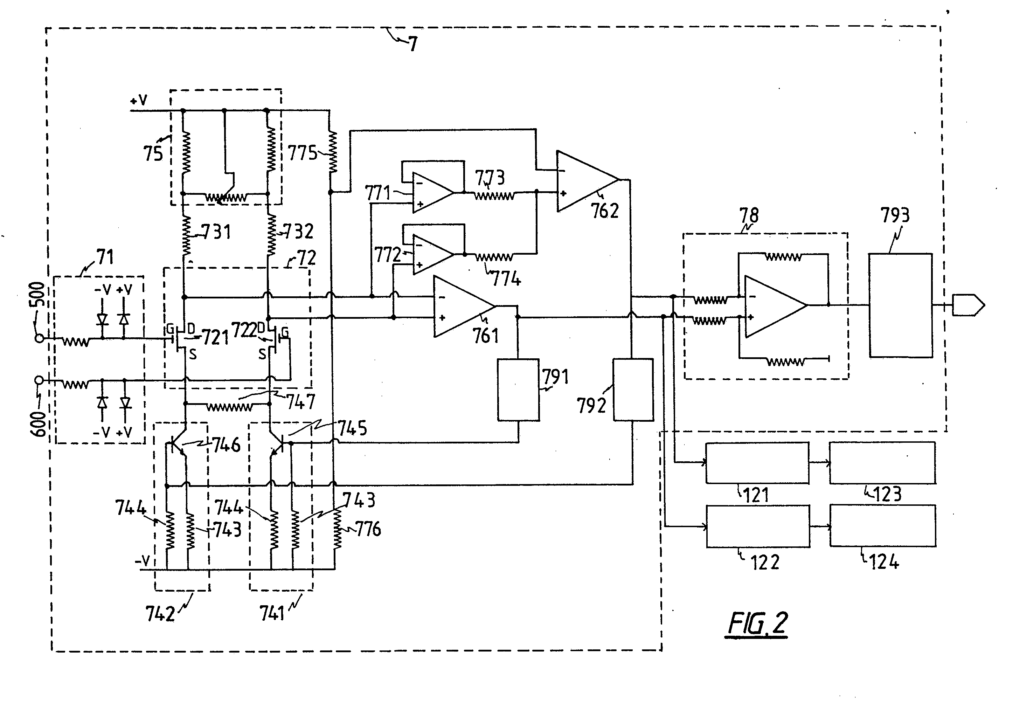

Dans un appareillage de captation multicanal suivant l'invention le circuit de conditionnement (7), tel que représenté à la figure 2, comporte une protection d'entrée (71) suivie d'un préamplificateur différentiel (72) formé de deux transistors (721 et 722) à effet de champs reliés respectivement aux électrodes (500 et 600). Le "drain" (D) de chacun de ces transistors (721 et 722) est relié respectivement à des résistance indentiques (731 et 732), reliées elles-mêmes à un circuit s'adjustement (75) du niveau zéro. Les sorties des transistors (721 et 722) à effet de champ de ce préamplifcateur (72) sont reliées d'une part aux entrées de l'amplificateur opérationnel (761) et d'autre part respectivement par l'intermédiaire des amplificateurs tampons (771 et 772) et des résistances de moyennage (773 et 774) à l'entrée positive du deuxième amplificateur opérationnel (762), qui reçoit de cette manière le niveau moyen du potentiel présent aux sorties des transistors (721 et 722) à effet de champ, tandis que l'entrée négative de ce deuxième amplificateur opérationnel (762) reçoit le niveau moyen du potentiel présent respectivement aux bornes positive et négative de l'alimentation par l'intermédiaire des résistances respectives (775 et 776).

Les sorties des amplificateurs opérationnels (761 et 762) sont raccordées par l'intermédiaire d'un circuit de décalage de tension (respectivement 791 et 792) en boucle de rétroaction aux sources de courant (741 et 742). Les circuits de décalage de tension (791 et 792) sont contitués, dans l'exemple décrit suivant l'invention, d'une simple résistance formant avec les résistances (743 et 744) un pont diviseur capable d'ajuster le courant de collecteur des transistors (745 et 746), à une valeur correspondant au point de fonctionnement optimum de courant de source des transistors (721 et 722) à effet de champ. Ce point de fonctionnement optimum dépend des applications de l'appareillage suivant l'invention, et peut-être, par exemple, le point du coefficent de température nul pour les transistors (721 et 722) à effet de champ.

L'entrée de chaque canal est donc constitué par un montage différentiel symétrique comportant un élément amplificateur à haute impédance d'entrée, les transistors (721 et 722) à effet de champ, pour chacun des deux entrées. Le niveau de fonctionnement, ou tension de référence de chacun des dits éléments amplificateurs est contrôlé par une boucle de rétroaction différente pour chacun d'entre eux. Pour l'un, le transistor (722) à effet de champ, cette boucle est commandée par l'amplitude du signal différentiel, venant de l'amplificateur opérationnel (761), tandis que l'autre (721) est commandée par la tension de mode commun, venant de l'amplificateur opérationnel (762).

Cette double boucle de rétroaction maintient les dits éléments amplificateurs à un niveau de fonctionnement relativement constant et parfaitement symétrique. Lorsqu'un signal, dans la méthode suivant l'invention de captation multicanale pour l'enregistrement digital de signaux électrophysiologiques lents et de faible amplitude apparaît au niveau d'une des électrodes (500 et 600) de mesure, la différence de potentiel transmise au premier amplificateur opérationnel (761) entraînera une modification en rétroaction négative de courant de source de transistor (722) à effect de champ. La même modifcation de potentiel imposera par l'intermédiaire du deuxième amplificateur opérationnel (762) une modification de sens contraire au niveau de l'autre transistor (721) à effet de champ. En conséquence le courant dans les deux transistors (721 et 722) à effet de champ sera maintenu près du niveau de départ. La petite modification qui subsiste dans chacun d'eux sera symétrique par rapport à ce point de fonctionnement.

Si les connections des électrodes (500 et 600) possèdent un potentiel d'électrode trop élevé, ou que, par suite d'un mauvais contact, ces entrées des électrodes (500 et 600) ne sont par reliées indirectement à la masse, les signaux sortant des amplificateurs opérationnels (761 et 762) seront très amples et pourront même atteindre la satura tion dans une tentative de contrôler le courant dans les transistor (721 et 722) à effet de champ.

Dans la méthode, suivant l'invention, de captaion multicanale, ces signaux, sortant des amplificateurs opérationnels (761 et 762), sont utilisés pour indiquer que de telles anomalies ont lieu aux électrodes (500 et 600). Les signaux font l'objet d'une comparaison dans des comparateurs analogiques (respectivement 121 ou 122) actionnant un aververtisseur ou un indicateur (123 ou 124) suivant que le signal provient de l'amplificateur opérationnel (762 ou 761), chaque fois que ce signal est trop élevé.

D'autres combinaisons sont possibles. Ces signaux d'avertissement peuvent être rassemblés par une porte - ou - en un seul avertisseur par canal, ou en un seul avertisseur pour tous les canaux tout en activant un décodeur avec un affichage numérique indiquant le canal ou l'électrode en cause. Ces affichages peuvent se faire au niveau de l'unité périphérique ou être transmis à l'unité centrale sous forme de signal de marquage d'événements, décrit plus loin.

Le signal venant du deuxième amplificateur opérationnel (762) représente un niveau de mode commun. Le signal venant du premier amplificateur opérationnel (761) contient le même signal de mode commun et le signal d'électrodes amplifié. Un troisième amplificateur (78) donne la différence entre ces deux signaux, soit le signal des électrodes amplifié.

La résistance (747), reliant les bornes correspondantes des transistors (721 et 722) à effet de champ, règle le gain du préamplificateur différentiel (72) constitué par ces deux transistors (721 et 722).

Le circuit de conditionnement (7), fig. 1, se termine par un filtre passe bas (793) essentiellement destiné à éviter les erreurs de type "aliasing" lors de la conversion analogique-numérique qui suivra. Le type de ce filtre et la fréquence de coupure dépendront de la plus petite fré quence d'échantillonnage qui est prévue de façon à ne laisser persister aucune composante significative du signal à une frequence égale ou supérieure à la moitié de cette fréquence d'echantillonnage.

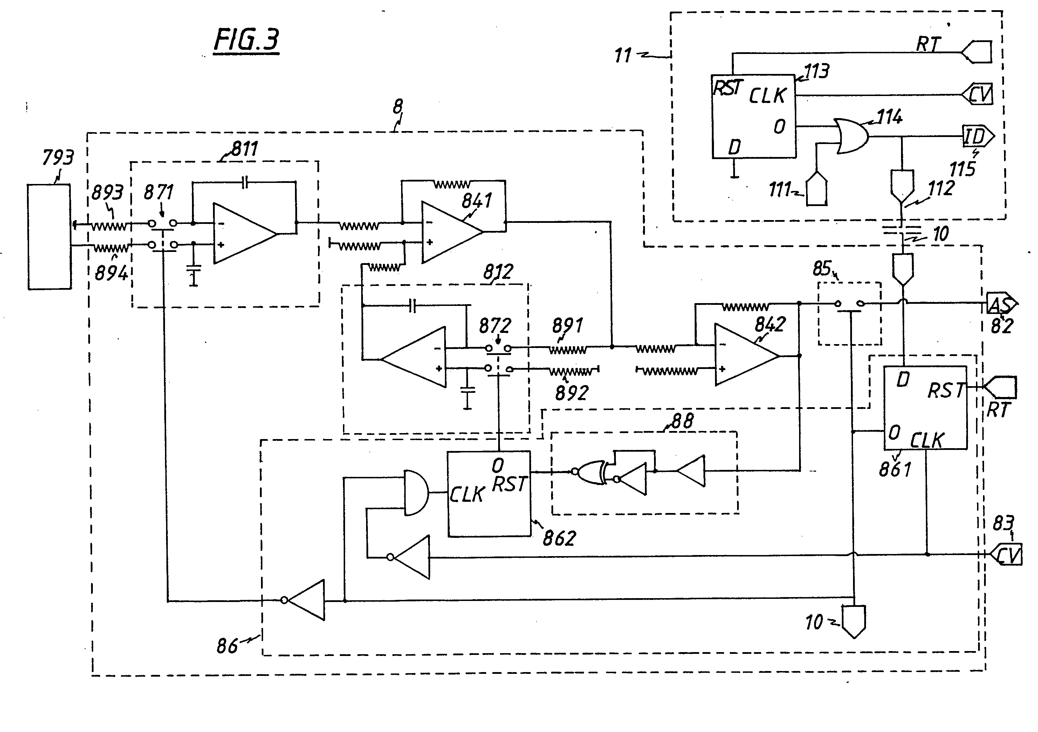

Le circuit d'échantillonnage différentiel et séquentiel (8) (fig. 1 et 3) contient un premier circuit (811) de type "échantillonage et maintien" dont l'entrée est reliée à la sortie du filtre passe-bas (793) (fig. 2 et 3) et dont la sortie est reliée à l'entrée négative de l'amplificateur différentiel (841), figure 3. Un deuxième circuit (812) de type "échantillonnage et maintien" est monté en rétroaction vers l'entrée positive du dit amplificateur différentiel (841). La sortie du dit amplificateur différentiel (841) passe, après amplification complémentaire dans l'amplificateur (842), à un interrupteur (85) contrôlé par un circuit logique (86) avant d'atteindre la sortie (82) du canal considéré: le signal (AS). Le dit circuit logique (86) contrôle aussi les deux circuits de type "échantillonnage et maintien" (811 et 812).

La sortie (82) du canal considéré, fig. 1, est reliée en étoile, par rapport aux sorties (82) des autres canaux, au circuit de transmission périphérique (9) qui contient un convertisseur analogique-numérique, décrit plus loin, qui effectue la conversion des échantillons analogiques venant des sorties (82) en échantillons numériques.

DAns la méthode, suivant l'invention, de captation multicanale, chaque canal présente à tour de rôle à sa sortie (82) un potentiel, le signal (AS), dont l'amplitude correspond à la différence entre le potentiel du signal enregistré lors d'une conversion analogique-numérique donnée et le potentiel du même signal lors de la conversion précédente du même canal.

Ceci est réalisé, dans l'appareillage de captation multicanal suivant l'invention, avec les deux circuits de type "échantillonnage et maintien" (811 et 812) et le circuit logique (86) de chaque circuit d'échantillonnage différentiel et séquentiel (8), figure 3. Ce circuit logique (86) est principalement contôlé par deux circuits à bascule (861 et 862). Le premier circuit à bascule (861) est remis à son état initial à l'allumage par un signal "reset" (RT) produit par exemple par un circuit RC relié à l'alimentation. Lorsque le dit convertisseur analogique-numérique qui suivra la sortie (82) effectue une conversion, l'entrée (83) reçoit un signal de conversion (CV) commun à tous les canaux et relié à l'entrée de déclanchement (CLK). L'entrée des données (D) du premier circuit à bascule (861) de chaque canal est relié à la sortie (0) du premier circuit à bascule (861) du canal précédent. Cette connection matérialise la connection circulaire (10, fig. 1 et 3) entre les canaux. Un canal est actif lorsque, et seulement lorsque, la sortie (O) de son premier circuit à bascule (861) est active et ferme l'interrupteur (85). Cette sortie (0) devient active lors du flanc montant du dit signal de conversion (CV) si l'entrée des données (D), et par conséquent la sortie (O) du premier circuit à bascule (861) du canal précédent, était active.

Lorsqu'un canal est actif, la sortie (O) du premier circuit à bascule (861) place le premier circuit de type "échantillonnage et maintien" (811) dans son état "maintien" en ouvrant son interrupteur (871). Cette sortie (O) active va déclancher le premier circuit à bascule (861) du canal suivant et le circuit échantillonnage différentiel et séquentiel (8) suivant sera donc activé dès la conversion suivante tandis que le canal actif actuellement ne le sera plus.

Le flanc descendant du signal de conversion (CV) va déclancher le second circuit à bascule (862) en activant son entrée (CLK). Le dit second circuit à bascule (862) contrôle le second circuit de type "échantillonnage et maintien" (812) et le met avec sa sortie (O) active dans son état "échantillonnage" en fermant son interrupteur (872). Le second circuit de type "échantillonnage et maintien" (812) effectue donc un échantillonnage pendant que le premier (811) est en position de "maintien". De la sorte, la sortie de l'amplificateur différentiel (841) passe à zéro. Dès le niveau zéro atteint, le circuit détecteur de zéro (88) remet le second circuit à bascule (862) dans son état initial par son entrée (RST).

Pendant tout ce temps, l'interrupteur (85) était fermé et la sortie du dit circuit d'échantillonnage (8) est restée connectée à la sortie (82) et donc au circuit de transmission périphérique (9, fig. 1), à son convertisseur analogique-numérique pour être plus précis.

Dès qu'un nouveau signal de conversion (CV) apparaît, la sortie (O) du premier circuit à bascule (861) devient inactive et l'interrupteur (85) est ouvert de façon à ce que seul le circuit d'échantillonnage (8) suivant soit connecté à la ligne du signal à convertir (AS). A ce moment, le circuit de type "échantillonnage et maintien" (811) retrouve son état d'échantillonnage, et, progreesivement, toute différence du potentiel d'entrée du système par rapport à l'état lors du dernier échantillonnage apparaît amplifiée à la sortie de l'amplificateur différentiel (841). Cette différence fera l'objet d'une nouvelle conversion numérique lors de la prochaine activation du dit canal.

Au moment où la sortie de l'amplificateur différentiel (841) est ramenée à zéro, le signal à la sortie des circuits de type "échantillonnage et maintien" (811 et 812) sont identiques pour autant que les quatre résistances contrôlant l'amplificateur différentiel (841) soient identiques. Si ce n'est pas le cas, la sortie de l'amplificateur différentiel (841) est annulée pour des valeurs des sorties des circuits (811 et 812) que l'on peut calculer suivant la théorie des amplificateurs opérationnels. Les résistances (891 et 892) permettent d'éviter les oscillations lors de la remise à zéro. Elles doivent cependant être suffisamment petites pour que le zéro soit atteint entre la fin de la conversion et la fin du temps "main tien" du circuit (811). En tenant compte du fait que l'interrupteur (871) du circuit d'échantillonnage (8) est ouvert pendant 1 temps sur les n temps d'échantillonnage pour interroger les n canaux du système complet, la valeur effective des résistances (893 et 894) vaudra la valeur réelle de la résistance multipliée par un facteur n/n-1.

Plusieurs types de circuits d' "échantillonnage et maintien" peuvent être utilisées en lieu et place des exemples différentiels représentés dans la figure 3. Pour certains types, des impulsions parasites provenant de leur signal logique de commande, devront être compensées dans le signal analogique. Ceci peut se faire par des impédances d'addition ou de soustraction aux entrées de l'amplificateur opérationnel (842). Cette compensation n'est pas représentée sur la figure.

Le circuit d'initialisation (11), figure 3, est inséré dans la connection circulaire (10) entre les circuits d'échantillonnage différentiel et séquentiel (8) (fig. 1) par son entrée (111) et sa sortie (112). Il contient essentiellement un circuit à bascule (113) qui contrairement aux circuits à bascule (861) des circuits d'échantillonnage (8), est activé par l'impulsion commune de "reset" (RT) au moment de l'allumage. Une première impulsion de commande peut ainsi être insérée dans la connection circulaire (10) par la porte - ou - (114). Dès le premier signal de conversion (CV), le circuit à bascule (113) est définitivement inactivé. Une sortie (115) permet, avec un signal (ID), d'indiquer le moment où, après un tour comlet de la connection circulaire (10), et donc de tous les canaux, le cycle va recommencer. Le signal (ID) (fig.1) est donc activé quand le canal qui précède le circuit d'initialisation (11) est actif. Un des canaux fournit donc un signal (ID) actif lorsque le canal correspondant est actif ce qui permettra ultérieurement d'identifier ce canal dans la transmission séquentielle de signaux des différents canaux vers l'unité centrale (200, fig. 1).

Le signal de conversion (CV) est dirigé vers le circuit d'échantillonnage différentiel et séquentiel (8) de tous les canaux et vers le circuit d'initialisation (11). Le signal (AS) venant du circuit d'échantillonnage différentiel et séquentiel (8) de tous les canaux convergeant en étoile est dirigé comme le signal (ID) venant du circuit d'initialisation (11) et le signal (EM) de marquage d'événement vers les entrée du cir cuit de transmission périphérique (9), figure 1.

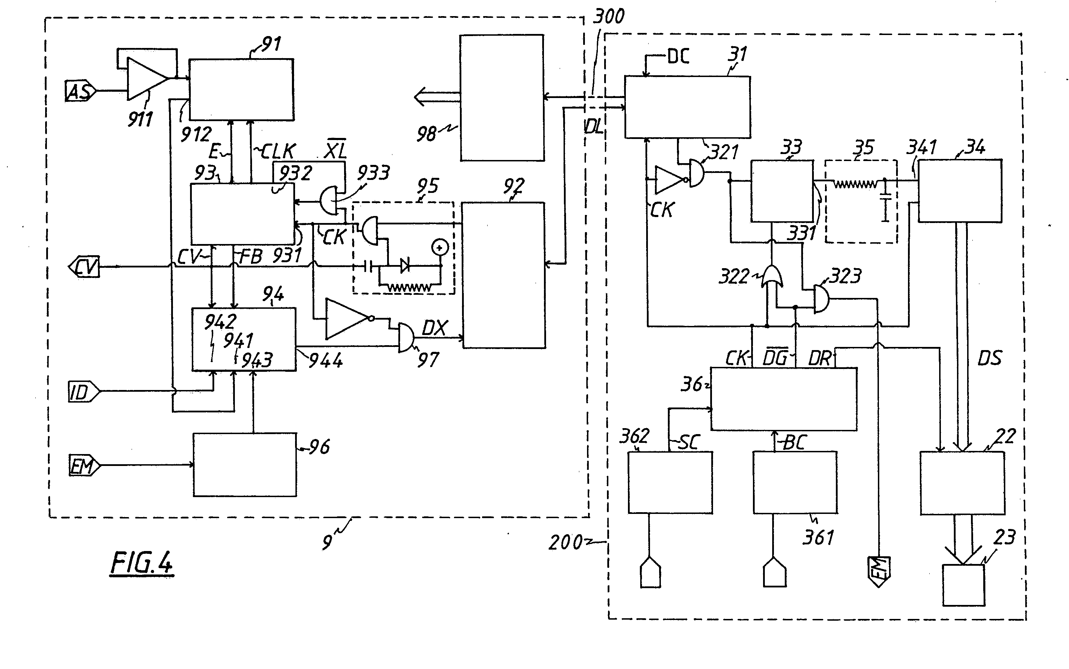

Le dit circuit de transmission périphérique (9), figure 4, contient principalement un convertisseur analogique-numérique (91) à sortie (912) série, un circuit de compteur de temps (93), un multiplexeur digital (94), un circuit de conditionnement (96), et un circuit d'isolation (92) qui relie le circuit de transmission périphérique (9) avec l'unité centrale (200) par l'intermédiaire d'une ligne (300) de liaison.

Le dit circuit de l'isolation (92), comprenant un optocoupleur ou autre élément d'isolation, reçoit des impulsions d'horloge provenant de l'unité centrale (200). Ceux-ci sont organisés par trains d'impulsions (CK) dont le fréquence des impulsions correspond à la vitesse de transmissions et dont la fréquence de répétition correspond à la fréquence de conversion analogique-numérique. Ces trains d'impulsions (CK) sont transmis à l'entrée (931) sensible au flanc descendant du circuit compteur de temps (93). La première impulsion du train, trouvant la sortie (932) à l'état haut produit une remise à l'état initial du circuit de compteur de temps (93) grâce à la porte - et - (933) avec le signal (XL) indiquant la longueur d'un train d'impulsions d'horloge. Le circuit compteur de temps (93) contrôle le convertisseur analogique-numérique (91) en utilisant les trains d'impulsions (CK) pour former les signaux de contrôle (E et CLK), dont (E) est le signal d'activation et (CLK) est le signal d'horloge; et fournit à une autre sortie le signal de conversion (CV) et le signal correspondant au cycle complet (FB) du premiers temps d'horloge qui servent à contrôler le multiplexeur digital (94).

L'entrée (941) du dit multiplexeur digital (94) est relié à la sortie (912) du convertisseur analogique-numérique (91), et son entrée (942) à la sortie (115) du circuit d'initialisation (11, fig. 3) pour le signal (ID) et son entrée (934) au circuit de conditionnement (96) qui reçoit le signal (EM) de marquage d'événement. Le multiplexeur digital (94) présente à sa sortie (944) le signal d'identification (ID) pendant le premier cycle d'horloge et la sortie série du convertisseur analogique-numérique (91), fournissant un échantillon du canal converti, pendant les autres cycles du train d'impulsions. Dès la fin du train d'impulsions (CK), la sortie (944) est reliée au signal (EM) conditionné par le circuit de conditionnement (96).

La sortie (944) du dit multiplexeur digital (94) est relié à une porte -et- (97) ouverte seulement lorsque le signal d'horloge, venant de l'unité centrale (200), est inactif. La sortie de la dite porte -et- (97) est reliée à l'entrée de l'émetteur de transmission de l'unité périphérique, compris dans le circuit d'isolation (92). Le signal (DX) formé par le multiplexeur digital (94) est donc transmis après l'isolation par optocoupleur, ou autre système d'isolation dans le circuit (92), vers l'unité centrale (200) à raison de un bit pendant chaque phase inactive entre les impulsions d'horloge.

La fin d'un signal de conversion (CV) bloque la réception des trains d'impulsion grâce au circuit réfractaire (95) ce qui permet de synchroniser l'unité centrale (200) et l'unité périphérique (100). La constante de temps du circuit réfractaire (95) doit être supérieure à un cycle de cloche pour permettre une synchronisation progressive. Cette synchronisation se fait dès le premier échantillonnage si la constante de temps est supérieure à la durée d'un train d'impulsions.

Le circuit de communication entre l'unité périphérique (100) et l'unité centrale (200) comporte un circuit émetteur et un circuit récepteur au niveau de l'unité périphérique (100) faisant partie du circuit d'isolation (92) et un autre circuit émetteur ainsi qu'un autre circuit récepteur au niveau de l'unité centrale (200) et faisant partie du circuit de liaison (31). Le circuit de transmission périphérique (9) ne peut pas activer le circuit de réception de la dite unité périphérique (100). Cette condition peut être remplie par exemple en inhibant alternativement le circuit émetteur et le circuit récepteur périphérique par une oscillaion à très haute fréquence par rapport aux impulsions de cloche. Dans le dit exemple, les circuits récepteurs doivent comporter un détecteur et un filtre permettant de retrouver les impulsions d'horloge et de données. La dite haute fréquence peut être générée par l'oscillateur du circuit émetteur et la détection peut être de type synchrône.

Le circuit récepteur de l'unité centrale (200) est inhibé pendant la phase active des impulsions d'horloge. Une seule et même ligne peut donc être utilisée pour transmettre les impulsions d'horloge de l'unité centrale (200) vers l'unité périphérique (100) et d'autre part, entrelacés entre les impulsions d'horloge, les données de l'unitée périphérique (100) vers l'unité centrale (200). La dite ligne de transmission peut être constituée par un système infra-rouge, un système ultrasonique ou un système électomagnétique si l'unité périphérique possède sa propre alimentation. La dite oscillation à très haute fréquence servant à inhiber alternativement le circuit émetteur et le circuit récepteur périphérique peut être utilisée pour générer les ultrasons ou les ondes électromagnétiques. La dite oscillation à très haute fréquence et la dite alimentation propre sont inutiles lorsqu'un cable à trois conducteurs, utilisés comme ligne de liaison (300), relie l'unité périphérique (100) et l'unité centrale (200). Un des trois conducteurs constitue la masse et peut constituer le blindage du cable. Le deuxième conducteur constitue la ligne de transmission du signl (DL) proprement dite. Le troisième conducteur transporte un courant d'alimentation et est reliée au primaire du convertisseur DC/DC (98) isolé. Bien qu'il soit possible d'utiliser une tension d'alimentation unique, des circuits d'alimentation distincte pour les parties analogiques et pour les parties digitales permettent de réduire le niveau de bruit dans des applications concernant des signaux de très faible amplitude.

Dans l'unité centrale (200) les données sont séparées des impulsions d'horloge par une porte (321) dont la sortie ne peut être activée par les données transmisses que pendant la phase inactive du signal d'horloge (CK). Le premier flanc montant des impulsions de données en logique positive ou le flanc descendant en logique négative déclanche le circuit à bascule (33), qui est remis à zéro par chaque impulsion d'horloge (CK) par la porte (322) et entre les trains d'impulsions d'horloge. Le dit déclanchement sur le flanc des données permet d'éviter les difficultés de synchronisation que provoquent les délais de transmission.

La sortie (331) du circuit à bascule (33) qui représente les données en série, est relié à l'entrée (341) du régistre à décalage (34) qui effectue la transformation des données série en parallèle. La longueur du dit registre à décalage (34) doit compter autant de bits que la longueur des mots de conversion numérique du convertisseur analogique-numérique (91), augmenté d'un bit (le premier) pour la transmission du signal indicateur de canal (ID). Le signal d'horloge (CK) commande aussi l'avancement des données dans le dit régistre à décalage (34). La même impulsion (CK) charge la sortie (331) du dit circuit à bascule (33) dans le dit registre à décalage (34) et d'autre part, remet à zéro la sortie (331) du dit circuit à bascule (33). Un élément de délai, comme le circuit de délai (35), est donc indispensable entre la sortie (331) du dit circuit à bascule (33) et l'entrée (341) du dit registrè à décalage (34) pour éviter les conflits de temps.

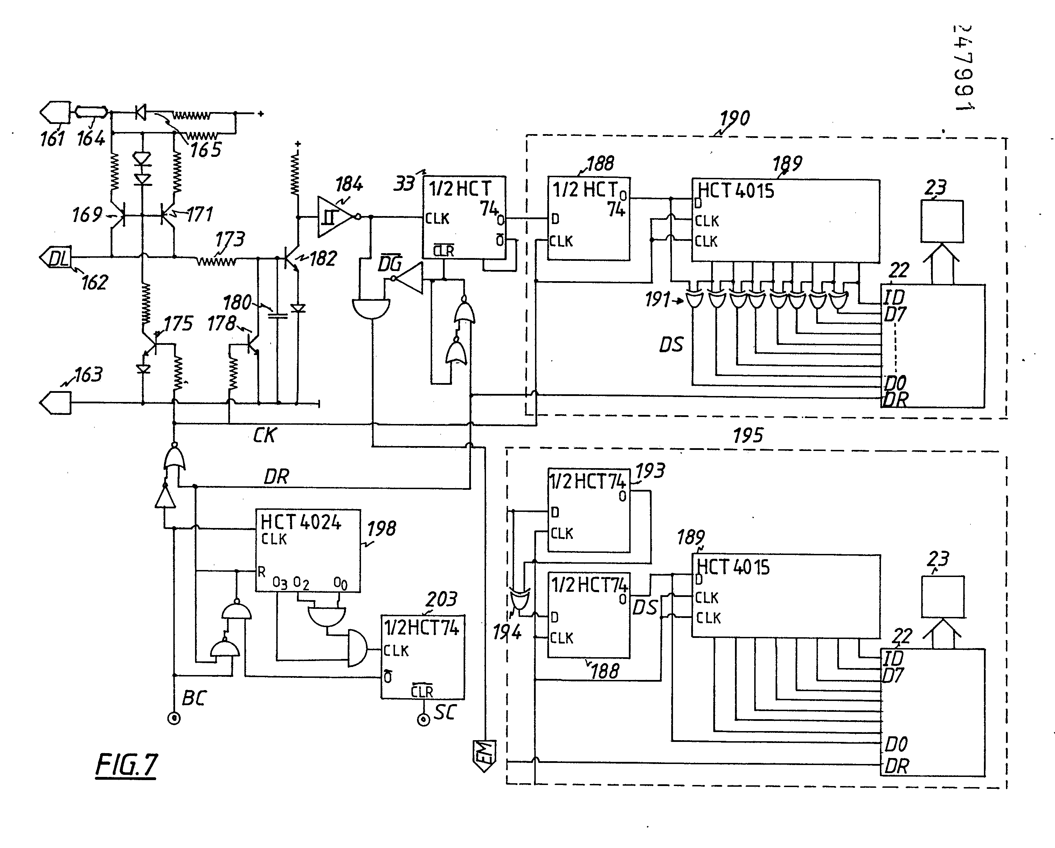

La figure 7 présente deux circuits alternatifs (190 et 195) pour la mise en parallèle des données qui permettent d'éviter le circuit de délai (35, fig. 4). Ces deux circuits (190 et 195) sont toujours précédés du circuit à bascule (33) déclanché par le premier flanc des données comme dans le premier exemple. Cette fois cependant le circuit à bascule (33) n'est plus remis à zéro à chaque signal de cloche mais il est monté en diviseur de fréquence par deux. Les données initiales sont alors reconstituées par des portes -ou exclusives- (191) aux sorties de registre à décalage (189) dans le circuit (190), soit par un deuxième circuit à bascule (193) et une seule porte -ou exclusive- (194) entre le circuit à bascule (33) et l'entrée du registre à décalage (189) dans le circuit (195). Dans les circuits (190 et 195), le registre à décalage de 9 bit est en fait constitué d'un circuit à bascule (188) suivi d'un registre à décalage de 8 bit (189).

La porte (323), figure 4, permet d'isoler le signal marqueur d'événements (EM) présent uniquement entre les trains d'impulsion.

Le circuit contrôleur de temps (36) est semblable à celui (93) du circuit de transmission périphérique (9) de l'unité périphérique (100). A partir d'un signal d'horloge continu (BC) définissant la vitesse de transmission, et d'un signal d'échantillonnage (SC) déterminant le rythme d'échantillonnage, ce circuit compteur de temps (36) génère les trains d'impulsions d'horloge (CK) transmis à l'unité périphérique (100) et contrôlant le dit registre à décalage (34). Le dit circuit compteur de temps (36) fournit également un signal (DR) actif entre les dits trains d'impulsions d'horloge (CK) et transmis ver l'interface (22) pour indiquer que les données du registre à décalage (34) sont disponibles. Le dit circuit compteur de temps (36) fournit un troisième signal (DG) actif pendant les périodes où aucune donnée normale n'est transmise. La porte (323) ouverte par le dit troisième signal (DG) permet de recueillir toute impulsion de marquage d'événements (EM) transmise par l'unité périphérique (100) en dehors des temps de transmission normaux.

Le signal marqueur d'événements peut répondre à de nombreuses applications dans le cadre de la présente invention. Le signal transmis peut être un signal modulé ou un simple marqueur de type tout ou rien. Le message peut concerner un état particulier de l'unité périphérique (100) comme par exemple une condition inacceptable à l'entrée telle que révélée par le comparateur analogiques (121 et 122, fig. 2). Le signal peut également représenter un message provenant de l'organisme étudié. Ainsi, dans le cadre de la mesure d'un temps de réaction, le signal marqueur d'événements peut très bien apporter la réponse à une stimulation d'un sujet examiné tandis que les électrodes des différents canaux de mesure enregistrent l'électroen céphalogramme.

Un signal marqueur d'événements différent peut être généré pour chaque canal d'entrée. Ces signaux sont alors rassemblés sur une ligne unique grâce à une porte logique ouverte par et pendant la durée du signal du canal correspondant dans la connection circulaire (10, fig. 3). Un multiplexage dans le temps est ainsi réalisé et le décodage se fait dans l'unité centrale (200) par la relation entre l'impulsion d'identification (ID) et le temps d'apparition d'un marqueur d'événements correspondant à un canal donné.

Le circuit générateur (361), fig. 4, peut produire le signal d'horloge continu (BC), qui représente la fréquence de base, à partir d'un oscillateur propre ou grâce à des diviseurs de fréquence à partir d'une horloge générale. Le circuit (362), qui fournit le signal d'échantillonnage (SC) déterminant le rythme d'échantillonnage, peut également varier en fonction des applications. Ainsi par exemple, un oscillateur construit à l'aide d'un circuit "phase locked loop" et d'un diviseur de fréquence permet de synchroniser une vitesse d'échantillonnage plus rapide à la fréquence du secteur, plus lente. Une telle synchronisation permet dans certaines situations de limiter les interférences du secteur lors de l'enregistrement de signaux de faible amplitude.

L'interface (22) du microprocesseur doit être construit suivant les exigences du microprocesseur utilisé. Dans sa version la plus simple, il comporte des registres tampons rendant les données accessible directement sur le "bus" (23) du microprocesseur grâce à un décodeur d'adresses.

Les données reçues par le microprocesseur représentent pour chaque canal les différences successives entre le niveau du signal au moment de deux temps d'échantillonnage consécutifs. Le niveau de départ du signal reste donc inconnu, ce qui n'a aucune importance dans le domaine d'application prévu pour l'exemple des figures (1 à 8).

Si, pour une application particulière, il était indispensable de connaître ce niveau de départ, un canal supplémentaire pourrait être consacré à la transmission directe, donc sans différenciation successive, du signal de sortie d'un circuit de conditionnement (7, fig. 1 et 2). Pour un circuit de conditionnement (7), il y aurait donc un échantillonneur de différences à gain élevé et un échantillonneur direct à gain réduit pour éviter les saturations. Une application typique de cette double transmission concerne l'enregistrement de réflexes psychogalvaniques. Dans ce type d'enregistrement, il convient de mesurer une variation lente et faible de potentiel superposé à un niveau constant nettement plus élevé. Dans la plupart des applications cependant, ce niveau de base ou de départ est dépourvu de signification et pourra donc être choisi arbitrairement par le microprocesseur comme étant par exemple nul.

Le potentiel de base superposé au signal peut parfois être sujet à des variations lentes appelées dérive. L'influence de la température et d'autres changements au niveau du circuit électronique d'entrée peut d'ailleurs ajouter une composante technique à cette dérive. Toute erreur constante dans le signal différentiel apparaîtra également lors de la reconstitution d'un signal non différencié comme une dérive. Pour éviter ce problème, le microprocesseur retire à chaque donnée reçue un coefficient destiné à compenser toutes les sources de dérive.

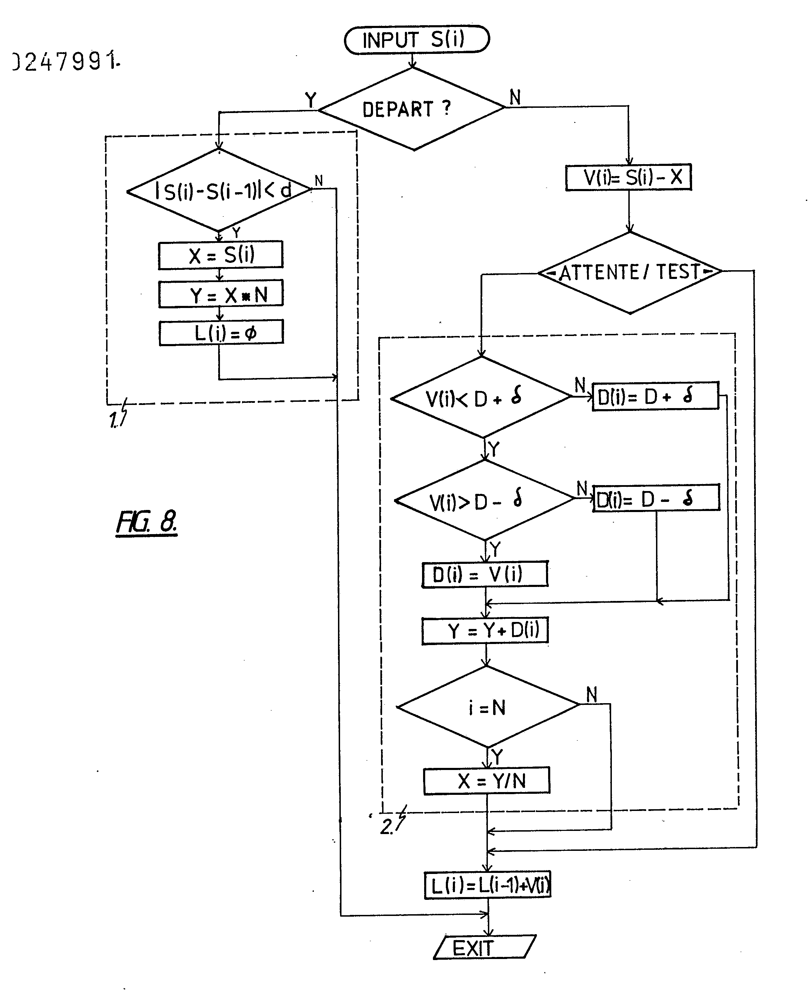

Au départ, ce coefficient de correction X est estimé, ce qui est représenté dans le cadre 1 de la figure 8, en sélectionnant deux échantillons différentiels S(i) consécutifs dont la valeur absolue de leur différence est inférieure à une valeur prédéterminée d et en égalisant ce coéfficient X à un échantillon différentiel S(i). La valeur d est donc une différence initialement admise entre deux échantillons. Les ajustements se font grâce à une estimation portant sur N échantillons successifs. N est donc le nombre d'échantillons inclus dans l'évolution de la dérive. Au départ, la dérive totale Y sur ces N échantillons est estimée comme étant égale à N x X. Le niveau du signal L(i) est initialisé sur une valeur arbitraire φ.

Par la suite, le coefficient de correction X sera ajusté continellement chaque fois que le système fonctionne sur un mode d'attente, ce qui est représenté dans le cadre 2, dans lequel V(i) est l'échantillon différentiel S(i) corrigé pour la dérive. Puisque en situation d'attente, la dérive devrait être nulle, il suffit d'ajouter à Y la valeur de N échantillons différentiels corrigés successifs pour obtenir une nouvelle estimation de Y et de X = Y/N. Afin d'éviter l'intervention de valeurs extrêmes non représentatives, les échantillons ajoutés à Y sont écrêtés à une amplitude de ± δ. δ est donc un écart maximal toléré pour la dérive et D est l'incrément de dérive. La procédure se termine par l'addition successive des valeurs corrigées V afin de reconstituer le signal de mesure L. Il s'entend que cette procédure s'applique de façon indépendante au signal de chacun des canaux.

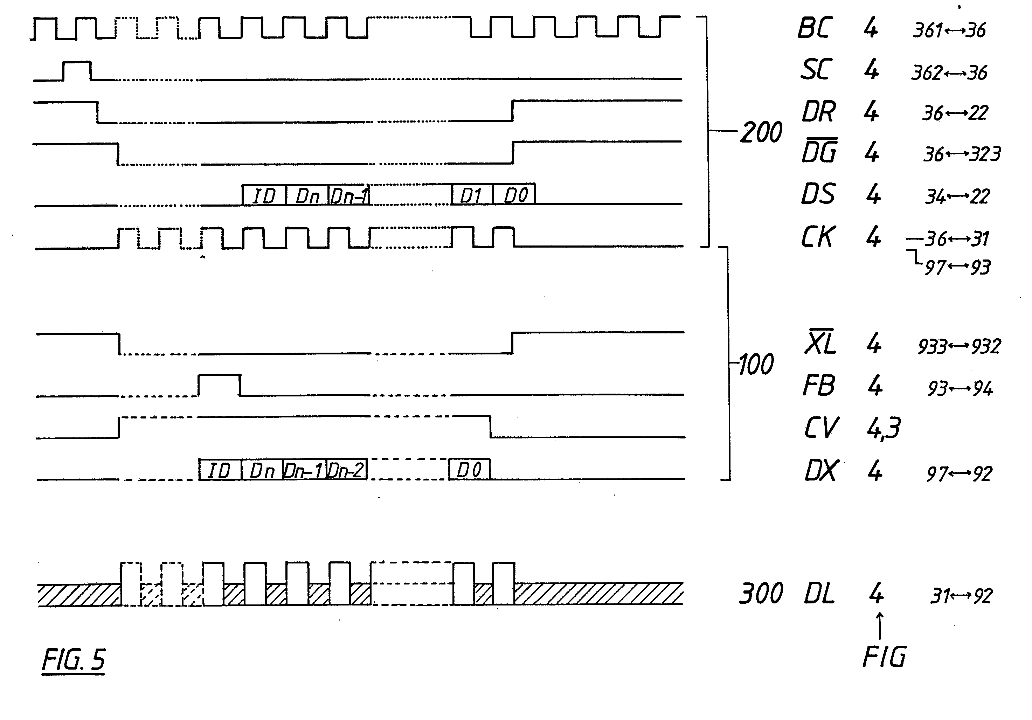

La figure 5 présente les relations temporelles des différents signaux de l'appareillage suivant l'invention. Dans l'unité centrale on a le signal d'horloge continu (BC) produit par le circuit générateur (361, fig. 4), le signal d'échantillonnage (SC) fournit par le circuit (362). De ces signaux (BC) et (SC) le compteur de temps (36) génère le signal (DR) transmis vers l'interface (22) et actif entre les trains d'impulsions d'horloge (CK), le signal (DG) aussi actif entre les trains d'impulsions d'horloge (CK) et activant entre autres la porte (323), et le troisième signal représentant les dits trains d'impulsions d'horloge (CK) transmis à l'unité périphérique (100) et transmis à l'entrée (931) du circuit compteur de temps (93) périphérique. Le signal (DS) représente un échantillon mis en parallèle par le registre à décalage (34). Le signal (DL) est celui qui est transmis entre l'unité centrale (200) et l'unité périphérique (100) sur un des conducteurs de la ligne de liaison (300).

Dans l'unité périphérique (100) on distingue le signal (XL) qui indique la longueur d'un train d'impulsions d'horloge (CK) venant de l'unité centrale (200) et qui remet le circuit de compteur de temps (93) à l'état initial. Le circuit de compteur de temps (93) fournit le signal (FB) qui correspond à un cycle complet du premier temps d'horloge et qui sert à contrôler le multiplexeur digital et le signal de conversion (CV, fig. 4 & 3). Le signal (DX) formé par le multiplexeur digital (94) est transmis vers l'unité centrale (200). Il faut remarquer que certains convertisseurs analogiques-numériques (91) exigent un certain nombre d'impulsions d'horloge initiales avant de commencer la conversion proprement dite. Ces cycles supplémentaires sont représentés en pointillé (3, fig. 5).

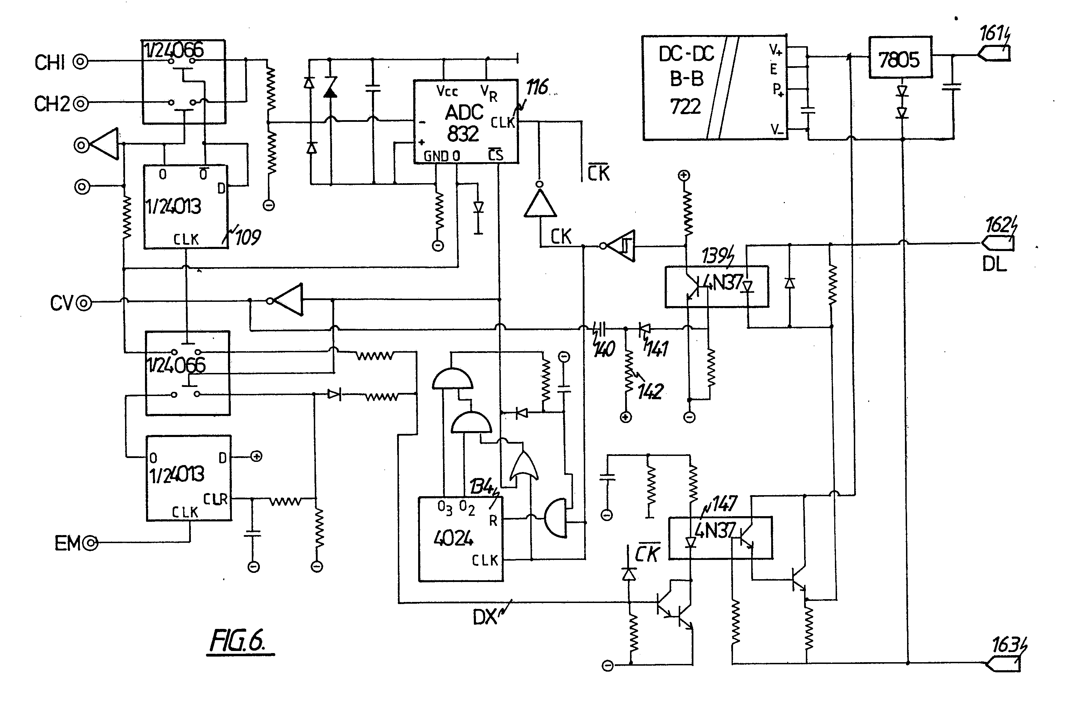

Les figures 6 et 7 représentent un exemple de réalisation d'un système à deux canaux (CH1 et CH2) permettant de simplifier le contrôle de la succession des canaux effectué par un seul circuit à bascule (109) à la place d'un circuit à bascule (861, fig. 4) pour chaque canal et le circuit à bascule (113, fig. 4). La figure 6 représente également le circuit de compteur de temps (116) et le convertisseur analogique-numérique (134), (respectivement 93 et 91 de la fig. 4). La transmission est assurée par deux optocoupleurs (139 et 147) montés de façon telle qu'ils partagent la même ligne de liaison (162) sans toutefois que la sortie de l'optocoupleur (147) ne puisse influencer l'entrée de l'optocoupleur (139). La porte (97) de la figure 4 est ici constituée par une simple diode commandée par l'inverse du signal CK. L'entrée du circuit Darlington activant l'optocoupleur de transmission est ainsi bloquée pendant la réception d'une impulsion CK. Le circuit réfractaire (95, fig. 4) est formé par le condensateur (140), la diode (141) et la résistance (142).

A l'autre extrémité de la ligne de liaison, figure 7, la transmission est contrôlée par les transistors (169 et 171), commandés par le transistor (175), qui transmettent les impulsions d'horloge sous forme d'implusions de courant devant activer l'optocoupleur (139, fig. 6). La porte (321, fig. 4) de blocage est représentée par le transistor (178) et la résistance (173). Le condensateur (180) ajoute un élément de filtre et le transistor (182) sert de tampon à l'inverseur (184). La diode LED (165) permet de constater au niveau de l'unité centrale que l'unité périphérique est bien connectée et que le fusible (164) est toujours en bon état. Le circuit compteur de temps (36, fig. 4) est ici représenté par le circuit (198) et les portes qui l'accompagnent ainsi que le circuit à bascule (203).

Dans l'exemple présenté, la connection (300, fig. 4) entre l'unité centrale (200, fig. 4) et l'unité périphérique (100, fig. 1) comporte les fils d'alimentation (161 et 163), dont le conducteur (163) est la masse, ainsi que la ligne de transmission (162). Cette connection pourrait être remplacé par une batterie au niveau de l'unité périphérique et une paire émetteur-récepteur infrarouge, ultrasonique ou électromagnétique à chaque extrémité de la ligne (162) qui serait supprimée. Du côté de l'unité périphérique il est indispensable d'ajouter un oscillateur à très haute fréquence, éventuellement commun au générateur d'émission et capable d'inhiber alternativement l'émetteur et le récepteur périphérique, afin d'éviter qu'ils n'interfèrent entre eux. Les deux récepteurs du système sont pourvus d'un détecteur, éventuellement de type synchrone, et d'un filtre capable d'éliminer la modulation à haute fréquence ainsi introduite afin de retrouver le signal initial.

Les signaux (BC, SC, DR, DG, DS, CK, DL, CV et DX) des figures 4 & 5 sont également indiqués sur les figures 6 & 7.

Les schémas de transmission prévus pourraient très bien dans le cadre de la présente invention, inclure un système de détection d'erreurs tels un bit de parité. Ce genre de précaution est cependant peu courant en biologie où une solution plus efficace pour améliorer la fiabilité de l'appareillage consiste à prélever plus d'échantillons que le minimum nécessaire.

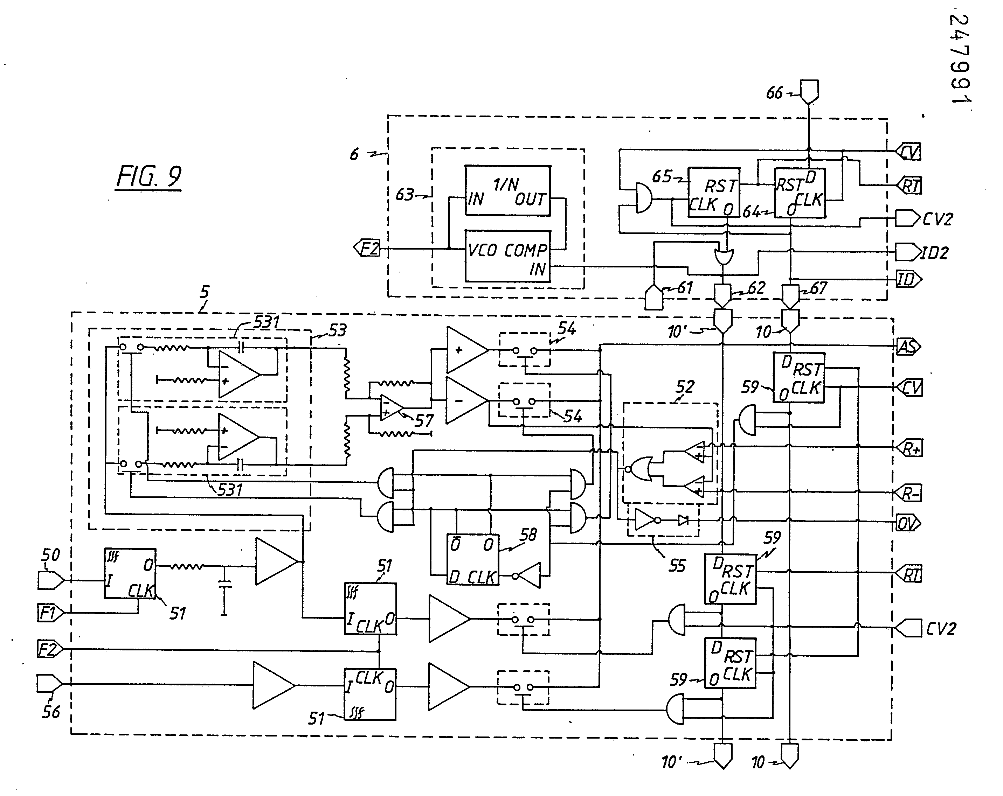

Un autre exemple de réalisation de la présente invention peut être décrit à l'aide de la figure 9. Cette figure peut être comparée à la figure 3. Elle comporte un circuit de dérivation (6) dans lequel un circuit à bascule d'initialisation (64) être insérée par les connections (66) et (67) dans une connection circulaire telle que décrite à la figure 1 sous le numéro (10) et appelée ici boucle primaire (10), par opposition à la boucle secondaire (10') connectée en (61) et (62). A chaque rotation complète de la boucle pimaire (10), l'élément de dérivation (6) dirige une impulsion d'échantillonnage (CV) vers le circuit à bascule d'initialisation (65) de la boucle secondaire (10'). Les impulsions d'échantillonnage ainsi obtenues sont appelées (CV2). La vitesse d'échantillonnage dans la boucle secondaire (10') sera donc nettement moins rapide que dans la boucle primaire (10) puisqu'un seul canal de la boucle secondaire (10') sera successivement activé par le système à chaque tour de la boucle primaire (10). Plusieurs boucles secondaires (10') peuvent être insérées et le nombre de canaux des boucles primaires (10) et secondaires (10') peut être choisi de façon à obtenir le rapport de fréquence d'échantillonnage souhaité pour chaque canal.

La boucle primaire (10) transmet un bit d'identification (ID) lors de l'activation du premier canal; de même, chaque boucle secondaire (10') transmet un bit (ID2) correspondant à son premier canal. Il est évident que des mots plus longs seront nécessaires lors de la transmission afin d'inclure les "bits" supplémentaires ainsi créés. Les différents signaux d'identification du premier bit peuvent être chacun également reliés à un multiplicateur de fréquence (63) de façon à fournir un signal de contrôle pour les filtres à "capacité commutée". Dans l'exemple donné, la fréquence (F2) est obtenue à partir de (ID2) et (F1) à partir de (ID). Cette dernière liaison n'a pas été représentée. Un tel arrangement présente l'avantage de voir la fréquence de coupure des filtres passe-bas (51) s'adapter à la fréquence d'échantillonnage sans nécessiter de modification des circuits de l'unité périphérique (100). L'unité centrale (200) pourra donc modifier les fréquences d'échantillonnage sans craindre d'introduire des erreurs de type "aliasing".

La figure 9 comporte également un exemple de circuit d'échantillonnage un peu différent de ce qui est représenté à la figure 3. En effet, dans le présent exemple, le circuit d'échantillonnage (5) contient trois canaux. Le premier, ayant pour entrée (50) la sortie de l'amplificateur (78) de la figure 2 et donc recevant le signal des électrodes amplifié, est inséré dans la boucle primaire (10). Les deux autres canaux sont insérés dans une boucle secondaire (10'). Chacun des trois canaux est contrôlé par un circuit à bascule (50) commandée par le signal de conversion (CV) pour le canal de la boucle primaire (10) et par le signal de conversion (CV2) pour les canaux de la boucle secondaire (10'). Ces systèmes de contrôle sont identiques à celui décrit dans la figure 3 et ils ne méritent donc aucune explication supplémentaire.

Aussi l'échantillonnage des différences entre les potentiels du signal enregistré lors de deux conversions analogiques, numériques consécutives est réalisé ici d'une façon différente. Pour le canal de la boucle primaire (10), le système d'échantillonnage (53) comporte deux circuits "échantillonneur et maintien" (531) identiques et activés alternativement grâce à un circuit à bascule (58) de sorte que leur signal de sortie correspond respectivement à la valeur du signal au temps d'échantillonnage actuel et au temps d'échantillonnage précédent. De nombreux types de circuits de types "échantillonneur et maintien", bien connus dans l'état actuel de la technique, peuvent convenir et les circuits présentés (53) ne sont que des exemples. Les sorties du système d'échantillonnage (53) sont connectées à un amplificateur différenciel (57). Ce dernier fournit les différences successives comparables à celles obtenues dans le circuit de la figure 3.

Les différences obtenues ont cependant une polarité inversée à chaque alternance d'échantillonnage et c'est la raison pour laquelle la porte de multiplexage de sortie, ayant la même fonction que la porte (85) de la figure 3, doit dans ce cas être dédoublée. Le signal étant inversé à l'une des entrées de cette double porte (54), la polarité peut être corrigée en activant alternativement l'une ou l'autre porte. Ce système d'échantillonnage à l'avantage de ne pas exiger l'utilisation du circuit de remise à zéro de la figure 3. Un tel circuit de remise à zéro impose en effet la présence d'un détecteur de zéro rapide (88, figure 3) et de boucles de rétro-action positive bien ajustées. Le circuit détecteur de zéro n'est cependant pas absolument indispensable dans l'exemple de la figure 3 et la remise à zéro peut être contrôlée directement par le signal de conversion, mais, dans ce cas, la précision du retour à zéro dépend du temps disponible. Ces difficultés n'existent pas dans l'exemple de la figure 9.

Le circuit de la figure 9 se distingue également par le comparateur (52). Ce comparateur (52) ouvre les interrupteurs du système d'échantillonnage (53) dès que le signal dépasse la référence positive (R+) ou devient inférieur à la référence negative (R-) utilisées par le convertisseur analogique-numérique. Cette ouverture résulte en un filtrage passe-bas plus sévère par le système d'échantillonnage (53) lui-même; ainsi, aucune erreur à long terme ne sera provoquée par un signal dont la pente est momentanément trop rapide. La sortie du comparatuer (52) est également reliée à un circuit (55) formant une fonction "ou" cablée avec les circuits semblables des autres canaux de sorte qu'un signal (OV) transmis à l'unité centrale (200) permet à cette dernière de réagir par une indication appropriée et, ou, en modifiant la vitesse d'échantillonnage.

Le signal direct tel qu'il entre dans le système d'échantillonnage (53) est transmis après amplification et filtrage appropriés par un des canaux de la boucle secondaire (10'). Les circuits de mise en forme sont classiques. Le filtrage peut être réalisé comme indiqué sur la figure 9 grâce à un filtre à "capacité commutée" (51) commandé par la fréquence (F2). Le deuxième canal de la boucle secondaire (10') est identique sauf son entrée (56) qui est reliée à la sortie de l'amplificateur (762, figure 2). Cette sortie fournit un signal proportionnel à la tension de mode commun si les ponts diviseurs (791), (743) et (792), (744) de la figure 2 ont été correctement ajustés.

Grâce à la triple voie du circuit (5) de la figure 9, il est possible de transmettre à l'unité centrale (200) l'amplitude du potentiel de mode commun, l'amplitude du signal direct et les différences d'amplitude successives du signal. Cette dernière valeur est fortement amplifiée et peut par sommations successives reconstituer le signal d'entrée avec une très grande dynamique. Des erreurs introduisent une dérive qui doit faire l'objet d'une correction simple (figure 8) ou plus complexe par exemple en modifiant les valeurs de d (figure 8) ou en utilisant le calcul statistique d'une droite de régression par la formule des moindres carrés.

Si l'unité centrale (200) reçoit la valeur du signal direct transmis avec une amplification beaucoup plus réduite et éventuellement avec un rythme beaucoup plus lent que les différences, il est possible d'utiliser cette valeur pour la comparer avec le résultat des sommations successive des différences. Toute erreur de la somme équivalente au bit le moins significatif du signal direct pourra faire l'objet d'une nouvelle estimation de la corection à appliquer aux échantillons des différences de façon à supprimer la dérive observée. Lorsque les corrections appliquées approchent une valeur idéale, ce type de correction interviendra plus rarement puisque les sommes successives donneront une image de plus grande résolution et un contenu plus riche en fréquence mais de valeur identique au signal direct.

La valeur du potentiel de mode commun également transmis à l'unité centrale (200) donne accès à l'information concernant le circuit d'entrée. Cette information peut être affichée ou enregistrée au niveau de l'unité centrale (200).

Les signaux (ID) peuvent être utilisés par l'unité centrale non seulement pour identifier le canal activé mais également pour calculer automatiquement le nombre de canaux installés dans l'unité périphérique et vérifier leur interrogation successivement grâce à un compteur des échantillons transmis entre deux signaux (ID). La même système peut s'appliquer à (ID2).

L'unité centrale (200) peut également déclencher des états différents de l'unité périphérique (100) en envoyant des impulsions d'horloge supplémentaires dans les trains d'impulsions. L'unité périphérique (100) possède un circuit réfractaire (95), (figure 4) limitant le nombre d'impulsions normalement présentes dans chaque train. Un tel circuit réfractaire pourrait d'ailleurs être réalisé grâce à un multiplicateur de fréquence alimenté par les impulsions de conversion (CV) et relié à un compteur. Toute impulsion d'horloge reçue pendant la période réfractaire peut être dérivée vers un circuit de contrôle capable d'activer différents états de l'unité périphérique (100) comme par exemple différents gains des amplificateurs ou différentes fréquences de coupure des filtres. Le déclenchement par un de ses états, d'un système classique de calibration et de mesure d'impédances des électrodes est une possi bilité. Un autre état peut induire un signal (RT) et ainsi provoquer un retour de toutes les boucles à leur état initial. Le déclenchement d'accessoires extérieurs au système est une autre application possible. Un autre état particulier de l'unité périphérique (100) pourrait consister à élever la connection de l'électrode de masse (400, figure 1) d'une petite valeur connue de potentiel par rapport à la masse du circuit d'entrée. Les valeurs de potentiel de mode commun et du signal obtenues lors de cette manoeuvre permettent de vérifier directement le bon fonctionnement de tous les circuits d'entrée et en particulier leur capacité de réjection du potentiel de mode commun.

Hormis la présence du compteur et décodeur des impulsions survenant pendant la période réfractaire ainsi que la présence de quelques portes supplémentaires, le circuit de transmission n'est pas différent de ce qui est représenté à la figure 4. Les circuits (93) et (94) en particulier doivent être adaptés au convertisseur analogique-numérique et à l'organisation des différentes boucles de canaux. Plusieurs combinaisons de compteurs et portes logiques et, ou, un microcontroleur et, ou, des mémoires de tupes PROM ou EPROM et, ou, circuits PLA peuvent trouver ici leur application.

La même remarque vaut pour le contrôle de la réception (figure 7). Certains protocoles de transmission permettent d'utiliser la sortie des circuits (193) et (194) comme contenant la dernière donnée transmise et ces sorties pourraient donc également être reliées à l'interface (22). La sortie de la bascule (33) peut être lue avant ou simultanément au flanc initial de la première impulsion (CK) d'un train d'impulsions pour indiquer la présence d'un signal entre les trains d'impulsions.

Lors de la transmission d'impulsions supplémentaires (telles que décrites plus haut) il est évidemment important d'éviter d'influencer le circuit de réception. Ces impulsions supplémentaires ne pourront donc pas apparaître sur les lignes CK et DR. Dans certaines applications, les signaux de contrôle CK et DR ainsi que la sortie de la bascule (188) ou (193) de la figure 7, suivant le code désiré, peuvent alimenter une sortie série lorsqu'une telle sortie est requise.

Dans les exemples présenté, les échantillons de signal sont digitalisés en 8 bit. Suivant les applications ce nombre de bit peut être différent.

Il est évident que suivant les applications envisagées, les circuits de la présente invention peuvent être réaliser en plusieurs classes de circuits telles bipolaire, CMOS, HCMOS, HCTMOS ou autres. De plus, l'organisation de l'appareillage peut dans le cadre de la présente invention se diviser en parties essentielles ou modules standardisés qui peuvent être réalisés sur microplaquettes par exemple. La construction d'un appareillage adapté à une application particulière revient alors à assembler simplement les modules nécessaires.

## Revendications

1. Méthode de captation multicanale pour l'enregistrement digital de signaux lents et de faible amplitude, plus en particulier de signaux électrophysilogiques captés au moyen d'électrodes (500 et 600) formant deux par deux les entrées de chaque canal d'une unité périphérique (100) de captation et transmis par une ligne (300) de liaison vers une unité centrale (200) d'enregistrement et de commande, caractérisée en ce que les canaux utilisés fournissent à tour de rôle un échantillon, représentant la différence d'amplitude du signal conditionné du canal en question tel qu'il se présente au moment où ledit canal est interrogé par rapport au moment de l'interrogation précédente, suivi d'une transmission sérielle des échantillons consécutifs.

2. Méthode de captation multicanale suivant la revendication 1, caractérisée en ce que le conditionnement des deux signaux d'entrée de chaque canal consiste, entr'autres, en une préamplification par un élément amplificateur propre à chaque entrée; le niveau de fonctionnement ou tension de référence des deux éléments amplificateurs est contôlé par une boucle de rétro-action différente pour chacun d'entre eux: la première boucle étant commandée par l'amplitude du signal différentiel des deux entrées, la deuxième boucle étant commandée par le signal de mode commun des deux entrées.

3. Méthode de captation multicanale suivant la revendication 2, caractérisée en ce que le niveau dudit signal différentiel des deux entrées de chaque canal et dudit signal de mode commun des deux entrées de chaque canal est directement surveillée par des comparateurs (121 et 122).

4. Méthode de captation multicanale suivant les revendications 1 et 2, caractérisée en ce que le signal conditionné de chaque canal utilisé représente la différence entre ledit signal de mode commun et ledit signal différentiel augmenté du mode commun.

5. Méthode de captation multicanale suivant une des revendications 1 à 4 caractérisée en ce que chaque canal produit, à part le signal conditionné de mesure, le signal de mode commun des deux entrées du canal considéré et/ou les signaux d'entrée directs du canal considéré et/ou les différences successives des valeurs des signaux d'entrée du canal considéré.

6. Méthode de captation multicanale suivant la revendication 5 caractérisée en ce que l'amplitude d'un ou plusieurs signaux d'un canal est limitée automatiquement par l'adaption d'un effet de filtre et dans laquelle l'apparition d'une telle adaption donne lieu à un autre signal du canal considéré.

7. Méthode de captation multicanale suivant la revendication 1, caractérisée en ce que des trains d'impulsions, provenant de l'unité centrale (200) d'enregistrement et de commande, commandent la fourniture à tour de rôle d'un échantillon des signaux utilisés des canaux utilisés, la transmission sérielle des échantillons consécutifs de l'unité périphérique (100) de captation vers l'unité centrale (200) d'enregistrement et de commande, et la reconversion en parallèle des échantillons.

8. Méthode de captation multicanale suivant la revendication 7, caractérisée en ce que l'unité périphérique (100) transmet successivement un échantillon numérisé de chacun desdits signaux au rythme de 1 bit au cours de chaque phase inactive des impulsions d'un train d'impulsions et chaque échantillon est accompagné d'un signal d'identification de canal par train d'impulsions.

9. Méthode de captation multicanale suivant la revendication 8, caractérisée en ce que la réception du côté de l'unité centrale (200) est inhibée pendant la phase active d'une impulsion d'un train d'impulsions et la transmission des échantillons numérisés du côté de l'unité périphérique (100) est inhibée pendant la réception d'une impulsion d'un train d'impulsions pourque la transmission des dits trains d'impulsions de l'unité centrale vers l'unité périphérique et la transmission des dits échantillons numérisés de l'unité périphérique vers l'unité centrale puissant repasser par seulement un seul conducteur.

10. Méthode de captation multicanale suivant une des revendications 3, 7, 8 et 9, caractérisée en ce que les temps séparant les trains d'impulsions sont utilisés pour transmettre de l'unité périphérique (100) ver l'unité centrale (200) un signal marqueur d'événement, codé ou non, signifiant un état particulier du système périphérique, tels que les résultats de la surveillance par les dits comparateurs (121 et 122), ou de l'organisme étudié.

11. Méthode de captation multicanale suivant une des revendications 7, 8, 9 et 10 caractérisée en ce que les échantillons numérisés reçus en série dans l'unité centrale (200) sont séparés des trains d'impulsions et des signaux marqueur d'événement et mis en parallèle, après quoi le signal capté par chaque canal est reconstruit par une sommation successive des échantillons successifs des canaux utilisés.

12. Méthode de captation multicanale suivant la revendication 7 caractérisée en ce qu'un nombre d'impulsions supplémentaires est additionné par l'unité centrale (200) aux trains d'impulsions envoyés de l'unité centrale vers l'unité périphérique (100) pourque l'unité centrale puisse induire différents états de l'unité périphérique.

13. Méthode de captation multicanale suivant une des revendications précédentes dans laquelle le signal reconstruit de chaque canal est corrigé pour la dérive provenant de l'influence de la température, des autres changements au niveau du circuit électronique ou variations lentes auxquelles les échantillons de chaque canal sont sujets, caractérisée en ce que chaque échantillon d'un canal est diminué d'un coefficient X qui est estimé au départ en prenant la valeur de l'un ou l'autre de deux échantillons consécutifs semblables à un degré prédéterminée d et qui est corrigée continuellement chaque fois que le système fonctionne sur un mode d'attente en additionnant la valeur limitée à une amplitude d'un écart maximal toléré et prédéterminé, de N échantillons et en divisant cette addition par le nombre N d'échantillons considérés; après quoi, les échantillons corrigés sont additionnés afin de reconstituer le signal de mesure de chaque canal.

14. Méthode de captation multicanale suivant une des revendications précédentes dans laquelle le signal reconstruit de chaque canal est corrigé pour la dérive, caractérisée en ce que la correction à appliquer est estimée par une comparaison épisodique du signal reconstruit avec le signal direct, moins ample et filtré.

15. Appareillage de captation multicanal pour l'enregistrement digital de signaux lents et de faible amplitude, plus en particulier de signaux électrophysiologiques, comportant une unité périphérique (100) de captation reliée d'un part à l'organisme examiné par une connection de masse (400) et plusieurs paires d'électrodes (500 et 600) formant les différents canaux de mesure et d'autre part à une unité centrale (200) d'enregistrement et de commande par une ligne de liaison (300), caractérisé en ce que chaque canal de l'unité périphérique possède son propre circuit d'échantillonnage (8) étant placé dans une connection circulaire (10) et relié en étoile par leurs sorties à l'entrée d'un circuit de numérisation des données analogiques, la sortie dudit circuit de numérisation étant reliée à l'entrée d'un circuit de transmission, les circuits d'échantillonnage (8) étant activés successivement par la combinaison d'un signal venant du circuit d'échantillonnage (8) précédent par la connection circulaire (10) et d'un signal de conversion (CV) commun à tous les circuits d'échantillonnage.

16. Appareillage de captation multicanal suivant la revendication 15 caractérisé en ce qu'un circuit d'initialisation (11) est inséré dans la connection circulaire (10); ce circuit d'initialisation (11) reçoit au début un signal de "reset" et le signal de la connection circulaire (10) après un tour complet de tous les circuits d'échantillonnage (8) des canaux utilisés de cette connection circulaire (10) et est relié au circuit de transmission (9) pour un signal d'identification (ID) indiquant qu'un desdits tours est complet.

17. Appareillage de captation multicanal suivant la revendication 15 dans lequel chaque circuit d'échantillonnage (8) est précédé d'un circuit de conditionnement (7) comportant un élément amplificateur (721 et 722) pour chaque électrode (500 et 600), caractérisé en ce que la sortie de chaque élément amplificateur (721 et 722) est relié par une boucle de retro-action propre à leur source de courant, la première boucle de retro-action fournissant le signal différentiel des deux électrodes, la deuxième boucle de retro-action fournissant le signal de mode commun des deux électrodes.

18. Appareillage de captation multicanal suivant la revenication 17 caractérisé en ce chaque boucle de retro-action est relié à un comparateur (121, 122) suivi d'un avertisseur (123, 124) ou indicateur.

19. Appareillage de captation multicanal suivant la revendication 15 dans lequel chaque canal peut inclure, à part le signal de mesure conditionné venant des signaux d'entrée des électrodes, un ou plusieures signaux additionnels, comme par exemple le signal de mode commun des deux électrodes, les signaux d'entrée directs, les différences successives des valeurs des signaux d'entrée, caractérisé en ce que chaque canal possède son propre circuit d'échantillonnage (8) placé dans une connection circulaire primaire (10), contrôlant l'activation successive des circuits d'échantillonnage des canaux et l'échantillonnage successif des différents signaux des canaux.

20. Appareillage de captation multicanal suivant la revendication 19 caractérisé en ce que les circuits d'échantillonnage sont placés dans une connection circulaire primaire (10) contrôlant l'activation successive des circuits d'échantillonnage (8) des canaux et l'échantillonnage successif d'un ou plusieurs signaux des canaux, et dans une ou plusieurs connections circulaires (10') secondaires contrôlant l'échantillonnage successif d'un ou plusieurs autres signaux des canaux et contrôlées par la connection circulaire primaire (10).

21. Appareillage de captation multicanal suivant la revendication 20 caractérisé en ce que les connections circulaires secondaires (10') sont insérées sur la connection circulaire primaire (10) de sorte que l'activation successive des signaux d'une connection circulaire secondaire (10') n'avance que d'un seul état pour chaque tour complet de la connection circulaire primaire (10).

22. Appareillage de captation multicanal suivant les revendications 15 et 19 caractérisé en ce que les signaux sont filtrés par un filtre à capacité commutée (51) dont la fréquence de coupure est adaptée à la frequence d'échantillonnage grâce à un circuit multiplicateur de fréquence (63).

23. Appareillage de captation multicanal suivant la revendication 15 caractérisé en ce que le circuit d'échantillonnage (5) consiste en deux circuits "d'échantillonnage et maintien" (531) identiques, montés en parallèle et activés successivement de sorte que la différence entre leur sorties fournisse après inversion successive de de polarité la différence de valeur entre le signal au temps actuel d'échantillonnage et la valeur du même signal au temps précédent.

24. Appareillage de captation dans lequel une ou plusieurs divisions de la méthode de captation multicanal suivant une des revendications 1 à 14 sont mises à l'ouvrage ou comprenent une ou plusieurs parties de l'appareillage de captation multicanal suivant une des revendications 15 à 22.

247991

FIG.1

FIG.2

FIG.3

0247991

FIG.4

)247991

| | | | |
|---|---|---|---|
| BC | 4 | 361↔36 | |
| SC | 4 | 362↔36 | |
| DR | 4 | 36↔22 | |
| $\overline{DG}$ | 4 | 36↔323 | |
| DS | 4 | 34↔22 | |
| CK | 4 | $-36↔31$ | |
| | | $97↔93$ | |
| $\overline{XL}$ | 4 | 933↔932 | |
| FB | 4 | 93↔94 | |
| CV | 4,3 | | |
| DX | 4 | 97↔92 | |
| 300 | DL | 4 | 31↔92 |

DS row: ID | Dn | Dn-1 | ... | D1 | D0

DX row: ID | Dn | Dn-1 | Dn-2 | ... | D0

200

100

FIG

FIG. 5

247991

FIG.6.

FIG.7

0247991.

FIG. 8.

FIG. 9

Office européen
des brevets

**RAPPORT PARTIEL
DE RECHERCHE EUROPEENNE**
qui selon la règle 45 de la Convention sur le brevet
européen est considéré, aux fins de la procédure ultérieure,
comme le rapport de recherche européenne

Numéro de la demande

EP    87 87 0074

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y. | US-A-3 910 257 (J.C. FLETCHER) | | A 61 B   5/00 |
| | * Abrégé; colonne 2, lignes 14-29, 37-62; colonne 3, lignes 28-30, 43-49,60-68; colonne 4, lignes 5-26; colonne 6, lignes 39-52, ligne 67 - colonne 7, ligne 5; figures 1-3,5 * | 1,7, 15,24 | A 61 B   5/04 <br> H 03 F   3/45 |
| A | | 2,3,6, 16,18 | |
| | --- | | |
| Y | DE-A-2 816 494 (COLLECTRON SYSTEM) | | |
| | * Pages 1,2: revendications 1,3-5; page 5, lignes 12-20; page 7, lignes 9-21; page 8, ligne 17 - page 9, ligne 4; figure * | 1,7, 15,24 | |
| A | | 4 | |
| | --- | | |
| A | GB-A-2 003 276 (A.E. KARZ) | | |
| | * Abrégé; page 3, lignes 20-36, 120-129; page 4, lignes 95-101; page 5, lignes 62-70; page 6, | 1,7,8, 10,15, 20,24 | |

**DOMAINES TECHNIQUES RECHERCHES (Int. Cl.4)**

A 61 B

H 03 F

### RECHERCHE INCOMPLETE ./.

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes: 15-24
Revendications ayant fait l'objet de recherches incomplètes: 1-14
Revendications n'ayant pas fait l'objet de recherches:
Raison pour la limitation de la recherche:

Méthode de traitement chirurgical ou
thérapeutique du corps humain ou animal
(voir art. 52(4) de la convention sur
le brevet européen).

| Lieu de la recherche | Date d'achèvement de la recherche | Examinateur |
|---|---|---|
| La Haye | 14-09-1987 | RIEB |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1505.1  03.82

| Office européen des brevets | RAPPORT PARTIEL DE RECHERCHE EUROPEENNE | Numéro de la demande |
|---|---|---|
| | | EP 87 87 0074 |

\- 2 \-

| DOCUMENTS CONSIDERES COMME PERTINENTS | | | CLASSEMENT DE LA DEMANDE (Int. Cl.⁴) |
|---|---|---|---|
| Catégorie | Citation du document avec indication, en cas de besoin, des parties pertinentes | Revendica-tion concernée | |
| | lignes 111-126; page 7, lignes 49-71; page 8, lignes 17-23,51-58; figures 2-4 * | | |
| | --- | | |
| A | US-A-3 885 552 (J.R. KENNEDY) | | |
| | * Abrégé; colonne 5, lignes 50-64; colonne 8, lignes 3-13,40-50; colonne 10, lignes 24-37; colonne 22, lignes 5-11,40-61; colonne 23, lignes 1-8; figures 1,2,10 * | 1,4,7, 8,10, 15,16, 20 | |
| | --- | | |
| A | US-A-3 757 778 (M.H. GRAHAM) | | DOMAINES TECHNIQUES RECHERCHES (Int. Cl.4) |
| | * Abrégé; colonne 1, lignes 37-50; colonne 2, lignes 48-55; colonne 4, lignes 1-17; colonne 5, lignes 33-65; colonne 6, lignes 5-43; colonne 7, lignes 13-22; figures 1,3,6,7 * | 2,16, 17 | |
| | --- | | |
| A | EP-A-0 161 154 (E.F.C.I.S.) | | |
| | * Abrégé; page 1, ligne 1 - page 2, ligne 24; figures 1,2 * | 2-5,17 | |
| | ---------------------- | | |

OEB Form 1505.3   06.78